(19) Europäisches Patentamt
European Patent Office
Office européen des brevets

(11) **EP 1 496 858 B1**

(12) # EUROPEAN PATENT SPECIFICATION

(45) Date of publication and mention of the grant of the patent:
**14.08.2013 Bulletin 2013/33**

(21) Application number: **03745781.9**

(22) Date of filing: **02.04.2003**

(51) Int Cl.:
***A61K 9/00*** *(2006.01)* ***A61K 31/46*** *(2006.01)*
***A61M 15/00*** *(2006.01)*

(86) International application number:
**PCT/EP2003/003431**

(87) International publication number:
**WO 2003/084502 (16.10.2003 Gazette 2003/42)**

(54) **INHALATION KIT COMPRISUNG INHALABLE POWDER OF TIOTROPIUM**

INHALATIONSSET MIT INHALIERBAREM TIOTROPIUM-PULVER

ENSEMBLE D'INHALATION COMPRENANT DE LA POUDRE DE TIOTROPIUM A INHALER

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **09.04.2002 EP 02007868**

(43) Date of publication of application:
**19.01.2005 Bulletin 2005/03**

(73) Proprietor: **Boehringer Ingelheim Pharma GmbH & Co.KG**
**55216 Ingelheim am Rhein (DE)**

(72) Inventor: **ZIERENBERG, Bernd**
**55411 BINGEN AM RHEIN (DE)**

(74) Representative: **Simon, Elke Anna Maria et al**
**Boehringer Ingelheim GmbH**
**Binger Strasse 173**
**55216 Ingelheim am Rhein (DE)**

(56) References cited:
**EP-A- 1 158 970**
**WO-A-00/28979**
**US-A- 4 524 769**
**US-A- 4 627 432**
**US-A- 5 590 645**
**US-B1- 6 182 655**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 496 858 B1

## Description

The invention relates to a method for the administration of powdered preparations containing tiotropium by inhalation.

Background to the invention

Tiotropium bromide is known from European Patent Application EP 418 716 A1 and has the following chemical structure:

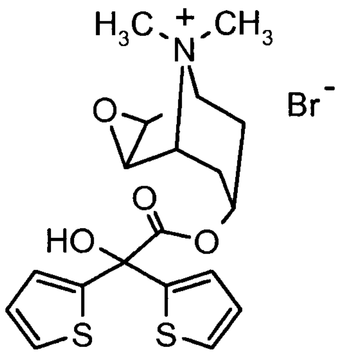

Tiotropium bromide is a highly effective anticholinergic with a long-lasting activity which can be used to treat respiratory complaints, particularly COPD (chronic obstructive pulmonary disease) and asthma. The term tiotropium refers to the free ammonium cation.

For treating the abovementioned complaints, it is useful to administer the active substance by inhalation. In addition to the administration of broncholytically active compounds in the form of metered aerosols and inhalable solutions, the use of inhalable powders containing active substance is of particular importance.

Prior art mentions examples for the administration of tiotropium containing pharmacetical compositions. As an example WO 00/28979 suggests powder formulations containing besides an active ingredient as for instance tiotropium bromide an amount of Mg-stearate. The said Mg-stearate is added to powder formulations, in particular to powder formulations for use in multiple dose dry-powder reservoir inhalers. EP 1158970 refers to combined preparations of tiotropium with formoterol and generally mentions a variety of generally applicable inhlation devices. Inhalation devices are further disclosed in US-A-5590645, US-A-4627432, US-A-4524769, US 5947118 and US D355029. With active substances which have a particularly high efficacy, only small amounts of the active substance are needed per single dose to achieve the desired therapeutic effect. In such cases, the active substance has to be diluted with suitable excipients in order to prepare the inhalable powder. Because of the large amount of excipient, the properties of the inhalable powder are critically influenced by the choice of excipient. When choosing the excipient its particle size is particularly important. As a rule, the finer the excipient, the poorer its flow properties. However, good flow properties are a prerequisite for highly accurate metering when packing and dividing up the individual doses of preparation, e.g. when producing capsules for powder inhalation or when the patient is metering the individual dose before using a multi-dose inhaler. It has also been found that the particle size of the excipient has a considerable influence on the proportion of active substance in the inhalable powder which is delivered for inhalation. The term inhalable proportion of active substance refers to the particles of the inhalable powder which are conveyed deep into the branches of the lungs when inhaled with a breath. The particle size required for this is between 1 and 10 $\mu$m, preferably less than 5 $\mu$m.

Finally, it has been found that the intended therapeutic effect upon the administration of a pharmaceutical composition via inhaltion can be decisively influenced by the inhalation device.

Accordingly, the aim of the invention is to provide for an inhalation kit comprising a tiotropium containing powder and an inhalation device, said kit being applicable in the method for administration mentioned before.

Detailed description of the invention

The invention relates to an inhalation kit consisting of an inhaler and an inhalable powder containing tiotropium in an amount of 0.001 to 5 %, in admixture with a physiologically acceptable excipient with an average particle size of between 10 to 500 $\mu$m, wherein the inhaler comprises a housing, containing two windows, a deck in which there are air inlet ports and which is provided with a screen secured via a screen housing, an inhalation chamber connected to the deck on which there is a push button provided with two sharpened pins and movable counter to a spring, a mouthpiece

which is connected to the housing, the deck and a cover via a spindle to enable it to be flipped open or shut, and three holes with diameters below 1 mm in the central region around the capsule chamber and underneath the screen housing and screen, and further characterized in that the said inhaler displays a flow resistance of about $0.01 - 0.1 \sqrt{kPa}$ min/l.

Of particular interest for inhalation kit according to the invention is an inhalable powder containing 0.01 to 2 %, preferably 0.04 to 0.8 %, more preferably 0.08 to 0.64 % tiotropium in admixture with a physiologically acceptable excipient.

More preferably in the inhalation kit according to the invention an inhalable powder containing 0.1 to 0.4 % tiotropium in admixture with a physiologically acceptable excipient is used.

By tiotropium is meant the free ammonium cation. The counter-ion (anion) may be chloride, bromide, iodide, methanesulphonate, para-toluenesulphonate or methyl sulphate. Of these anions, the bromide is preferred.

Accordingly, the inhalation kit according to the present invention preferably contains inhalable powders which contain tiotropium in form of tiotropium bromide in an amount of 0.0012 to 6.02 %, in admixture with a physiologically acceptable excipient. Of particular interest for the inhalation kit according to the invention is an inhalable powder containing 0.012 to 2.41 %, preferably 0.048 to 0.96 %, more preferably 0.096 to 0.77 % tiotropium bromide in admixture with a physiologically acceptable excipient.

More preferably in the inhalation kit according to the invention an inhalable powder containing 0.12 to 0.48 % tiotropium bromide in admixture with a physiologically acceptable excipient is used.

Tiotropium bromide is, depending on the choice of reaction conditions and solvents, obtainable in different crystalline modifications. Most preferred according to the invention are those powder preparations, that contain tiotropium in form of the crystalline tiotropium bromide monohydrate. Accordingly, the powdered preparations obtainable according to the invention preferably contain 0.0012 to 6.25 % crystalline tiotropium bromide monohydrate in admixture with a physiologically acceptable excipient is administered. Of particular interest for the inhalation kit according to the invention is an inhalable powder containing 0.0125 to 2.5 %, preferably 0.05 to 1 %, more preferably 0.1 to 0.8 % crystalline tiotropium bromide monohydrate in admixture with a physiologically acceptable excipient.

More preferably in the inhalation kit according to the invention an inhalable powder containing 0.12 to 0.5 % crystalline tiotropium bromide monohydrate in admixture with a physiologically acceptable excipient is used.

Examples of physiologically acceptable excipients which may be used to prepare the inhalable powders applicable according to the invention include, for example, monosaccharides (e.g. glucose or arabinose), disaccharides (e.g. lactose, saccharose, maltose), oligo- and polysaccharides (e.g. dextrane), polyalcohols (e.g. sorbitol, mannitol, xylitol), salts (e.g. sodium chloride, calcium carbonate) or mixtures of these excipients with one another. Preferably, mono- or disaccharides are used, while the use of lactose or glucose is preferred, particularly, but not exclusively, in the form of their hydrates, preferably in the form of their monohydrates.

In the inhalation kit according to the invention the average particle size of the physiologically acceptable excipient is between 10 to 500 μm, preferably between 15 to 200 μm, more preferably between 20 to 100μm. If not otherwise emphazised the term average particle size according to the invention is to be understood as the Mass Median Aerodynamic Diameter (MMAD). Methods for the determination thereof are known in the art.

Besides the coarser particle fraction of the excipient mentioned hereinbefore, the excipient can optionally additionally contain a specifically added fraction of excipient of finer particle size. This finer particle size fraction is characterized by an average particle size of 1 to 9 μm, preferably 2 to 8 μm, more preferably 3 to 7 μm.

If a finer particle fraction is present the proportion of finer excipient in the total amount of excipient is 1 to 20 %, preferably 3 to 15%, more preferably 5 to 10%. When reference is made to a mixture within the scope of the present invention, this always means a mixture obtained by mixing together clearly defined components. Accordingly, when an excipient mixture of coarser and finer excipients is mentioned, this can only denote mixtures obtained by mixing a coarser excipient component with a finer excipient component.

The percentages given within the scope of the present invention are always percent by weight.

In the inhalation kit according to the invention the inhalable powders mentioned hereinbefore may effeciently be adminstered using the inhaler depicted in figure 1 and described in more detail hereinbelow that is characterized by a specific flow resistance (R).

The flow resistance of inhalers can be calculated via the following formula:

$$V = \frac{1}{R} \cdot \sqrt{p}$$

wherein

v is the volumetric flow rate (l/min),
p is the pressure drop (kPa), and
R is the flow resistance.

In the inhalation kit according to the invention the flow resistance R characterising the inhaler is in a range of about $0.01 - 0.1\ \sqrt{kPa}$ min/l preferably in the range of about $0.02 - 0.06\ \sqrt{kPa}$ min/l.

Accordingly, the invention relates to an inhalation kit consisting of an inhalable powder containing tiotropium in an amount of 0.001 to 5 % in admixture with a physiologically acceptable excipient with an average particle size of between 10 to 500 μm, and an inhaler displaying a flow resistance of about $0.01 - 0.1\ \sqrt{kPa}$ min/l.

In another embodiment, the invention relates to a method for the treatment of airway diseases, particularly COPD (chronic obstructive pulmonary disease) and asthma, characterized in that an inhalable powder containing tiotropium in an amount of 0.001 to 5 %, in admixture with a physiologically acceptable excipient with an average particle size of between 10 to 500 μm, is administered via inhalation by an inhaler displaying a flow resistance of about $0.01 - 0.1\ \sqrt{kPa}$ .min/l.

In another embodiment the invention relates to the use of the inhaler for the administration of a tiotropium containing inhalable powder via inhalation, characterised in that the inhalable powder contains tiotropium in an amount of 0.001 to 5 % in admixture with a physiologically acceptable excipient with an average particle size of between 10 to 500 μm, and further characterized in that the said inhaler displays a flow resistance of about $0.01 - 0.1\ \sqrt{kPa}$ min/l.

In yet another embodiment the invention relates to an inhalation kit consisting of the inhaler exemplified in figure 1 displaying a flow resistance of about $0.01 - 0.1\ \sqrt{kPa}$ min/l min/l and an inhalable powder containing tiotropium in an amount of 0.001 to 5 % in admixture with a physiologically acceptable excipient with an average particle size of between 10 to 500 μm.

For the administration of tiotropium containing powders by inhalation by means of the inhaler according to figure 1, it is required to fill appropriate amounts of the powder into capsules. Methods for filling powders into capsules are known in the art. The inhaler according to figure 1 is characterised by a housing 1 containing two windows 2, a deck 3 in which there are air inlet ports and which is provided with a screen 5 secured via a screen housing 4, an inhalation chamber 6 connected to the deck 3 on which there is a push button 9 provided with two sharpened pins 7 and movable counter to a spring 8, a mouthpiece 12 which is connected to the housing 1, the deck 3 and a cover 11 via a spindle 10 to enable it to be flipped open or shut and three holes 13 with diameters below 1 mm in the central region around the capsule chamber 6 and underneath the screen housing 4 and screen 5.
The main air flow enters the inhaler between deck 3 and base 1 near to the hinge. The deck has in this range a reduced width, which forms the entrance slit for the air. Then the flow reverses and enters the capsule chamber 6 through the inlet tube. The flow is then further conducted through the filter and filter holder to the mouthpiece. A small portion of the flow enters the device between mouthpiece and deck and flows then between filterholder and deck into the main stream. Due to production tolerances there is some uncertainty in this flow because of the actual width of the slit between filterholder and deck. In case of new or reworked tools the flow resistance of the inhaler may therefore be a little off the target value. To correct this deviation the deck has in the central region around the capsule chamber 6 and underneath the screen housing 4 and screen 5 three holes 13 with diameters below 1 mm. Through these holes 13 flows air from the base into the main air stream and reduces such slightly the flow resistance of the inhaler. The actual diameter of these holes 13 can be chosen by proper inserts in the tools so that the mean flow resistance can be made equal to the target value.

In another preferred embodiment the invention relates to the use of the inhaler according to figure 1, comprising a housing, containing two windows,
a deck in which there are air inlet ports and which is provided with a screen secured via a screen housing, an inhalation chamber connected to the deck on which there is a push button provided with two sharpened pins and movable counter to a spring;
a mouthpiece which is connected to the housing, the deck and a cover via a spindle to enable it to be flipped open or shut, and three holes with diameters below 1 mm in the central region around the capsule chamber and underneath the screen housing and screen,
for the administration of an inhalable powdered containing tiotropium in an amount of 0.001 to 5 %, in admixture with a physiologically acceptable excipient with an average particle size of between 10 to 500 μm.

In yet another preferred embodiment the invention relates to an inhalation kit consisting of an inhalable powdered

containing tiotropium in an amount of 0.001 to 5 %, in admixture with a physiologically acceptable excipient with an average particle size of between 10 to 500 μm, and the inhaler according to figure 1, comprising a housing, containing two windows, a deck in which there are air inlet ports and which is provided with a screen secured via a screen housing, an inhalation chamber connected to the deck on which there is a push button provided with two sharpened pins and movable counter to a spring, a mouthpiece which is connected to the housing, the deck and a cover via a spindle to enable it to be flipped open or shut,
and three holes with diameters below 1 mm in the central region around the capsule chamber and underneath the screen housing and screen.

The following Examples serve to illustrate the present invention further without restricting its scope to the embodiments provided hereinafter by way of example.

**Starting materials**

As a starting material for the synthesis of crystalline tiotropiumbromide monohydrate tiotropiumbromide obtained according to the disclosure of European patent application EP 418 716 A1 is be used.

Preparation of crystalline tiotropium bromide monohydrate:

15.0 kg of tiotropium bromide as obtained according to the methods disclosed in EP 418 716 A1 are added to 25.7 kg of water in a suitable reaction vessel. The mixture is heated to 80-90°C and stirred at constant temperature until a clear solution is formed. Activated charcoal (0.8 kg), moistened with water, is suspended in 4.4 kg of water, this mixture is added to the solution containing the tiotropium bromide and rinsed with 4.3 kg of water. The mixture thus obtained is stirred for at least 15 min at 80-90°C and then filtered through a heated filter into an apparatus which has been preheated to an outer temperature of 70°C . The filter is rinsed with 8.6 kg of water. The contents of the apparatus are cooled at 3-5°C every 20 minutes to a temperature of 20-25°C. The apparatus is further cooled to 10-15°C using cold water and crystallisation is completed by stirring for at least one hour. The crystals are isolated using a suction drier, the crystal slurry isolated is washed with 9 litres of cold water (10-15°C) and cold acetone (10-15°C). The crystals obtained are dried in a nitrogen current at 25°C over 2 hours.
Yield : 13.4 kg of tiotropium bromide monohydrate (86 % of theory)

The crystalline tiotropium bromide monohydrate thus obtained is micronised by known methods, to bring the active substance into the average particle size which meets the specifications according to the invention.

The DSC diagram of crystalline tiotropium bromide monohydrate shows two characteristic signals. The first, relatively broad, endothermic signal between 50-120°C can be attributed to the dehydration of the tiotropium bromide monohydrate to produce the anhydrous form. The second, relatively sharp endothermic peak at 230 ± 5°C can be put down to the melting of the substance. These data were obtained using a Mettler DSC 821 and evaluated with the Mettler STAR software package. These data, like the other values given in the above Table, were obtained at a heating rate of 10 K/min.

The crystalline tiotropium bromide monohydrate thus obtained was characterised by IR spectroscopy. The data was obtained using a Nicolet FTIR spectrometer and evaluated with the Nicolet OMNIC software package, version 3.1. The measurement was carried out with 2.5μmol of tiotropium bromide monohydrate in 300 mg of KBr. Table 1 shows some of the essential bands of the IR spectrum.

Table 1: Attribution of specific bands

| Wave number ($cm^{-1}$) | Attribution | Type of oscillation |
|---|---|---|
| 3570, 410 | O-H | elongated oscillation |
| 3105 | Aryl C-H | elongated oscillation |
| 1730 | C=O | elongated oscillation |
| 1260 | Epoxide C-O | elongated oscillation |
| 1035 | Ester C-OC | elongated oscillation |
| 720 | Thiophene | cyclic oscillation |

The crystalline tiotropium bromide monohydrate was characterised by X-ray structural analysis. The measurements of X-ray diffraction intensity were carried out on an AFC7R- 4-circuit diffractometer (Rigaku) using monochromatic copper $K_{\alpha}$ radiation. The structural solution and refinement of the crystal structure were obtained by direct methods (SHELXS86 Program) and FMLQ-refinement (TeXsan Program). The X-ray structural analysis carried out showed that crystalline tiotropium bromide hydrate has a simple monoclinic cell with the following dimensions: a = 18.0774 Å, b = 11.9711 Å, c = 9.9321 Å, $\beta$ = 102.691°, V = 2096.96 Å$^3$.

**Apparatus**

The following machines and equipment, for example, may be used to prepare the inhalable powders according to the invention:

Mixing container or powder mixer: Gyrowheel mixer 200 L; type: DFW80N-4; made by: Messrs Engelsmann, D-67059 Ludwigshafen.

Granulating sieve: Quadro Comil; type: 197-S; made by: Messrs Joisten & Kettenbaum, D-51429 Bergisch-Gladbach.

The following examples provide for inhalable powder mixtures applicable according to the invention.

**Example 1:**

5.2 kg of glucose monohydrate for inhalation (average particle size 25μm) are used as the excipient. 22.5 g crystalline tiotropiumbromide monohydrate (micronised; average particle size 1 - 3.5 μm) are used as the active ingredient.

The aforementioned components are sieved in in alternate layers of lactose monohydrate in batches of about 200 g and crystalline tiotropiumbromide monohydrate in batches of about 1g. The ingredients sieved in are then mixed together (mixing at 900 rpm).

According to the invention preferably 5.2225 mg of the aforementioned powder are delivered per dose.

**Example 2:**

5.4775 kg of lactose monohydrate for inhalation (average particle size 25μm) are used as the excipient. 22.5 g crystalline tiotropiumbromide monohydrate (micronised; average particle size 1 - 3.5 μm) are used as the active ingredient.

The aforementioned components are sieved in in alternate layers of lactose monohydrate in batches of about 200 g and crystalline tiotropiumbromide monohydrate in batches of about 1g. The ingredients sieved in are then mixed together (mixing at 900 rpm).

According to the invention preferably 5.5 mg of the aforementioned powder are delivered per dose.

**Example 3:**

1.1: Excipient mixture:

5.203 kg of lactose monohydrate for inhalation (average particle size 25 μm) are used as the coarser excipient component. 0,27 kg of lactose monohydrate (5μm) are used as the finer excipient component. In the resulting 5,473 kg of excipient mixture the proportion of the finer excipient component is 5%.

The aforementioned components are sieved in in alternate layers of lactose monohydrate (25 μm) in batches of about 200 g and lactose monohydrate (5 μm) in batches of about 10g. The ingredients sieved in are then mixed together (mixing at 900 rpm).

1.2: Final mixture:

To prepare the final mixture, 5,473 kg of the excipient mixture (1.1) and 22.5 g crystalline tiotropiumbromide monohydrate (micronised; average particle size 1 - 3.5 μm) are used. The content of active substance in the resulting powder is 0.4%.

The aforementioned components are sieved in in alternate layers of excipient mixture (1.1) in batches of about 200 g and crystalline tiotropiumbromide monohydrate in batches of about 1g. The ingredients sieved in are then mixed together (mixing at 900 rpm).

According to the invention preferably about 5.5 mg of the aforementioned powder are delivered per dose.

**Claims**

1. Inhalation kit consisting of an inhaler and an inhalable powder containing tiotropium in an amount of 0.001 to 5 %, in admixture with a physiologically acceptable excipient with an average particle size of between 10 to 500 μm, wherein the inhaler comprises a housing, containing two windows, a deck in which there are air inlet ports and which is provided with a screen secured via a screen housing, an inhalation chamber connected to the deck on which there is a push button provided with two sharpened pins and movable counter to a spring, a mouthpiece which is connected to the housing, the deck and a cover via a spindle to enable it to be flipped open or shut, and three holes with diameters below 1 mm in the central region around the capsule chamber and underneath the screen housing and screen, and further **characterized in that** the said inhaler displays a flow resistance of about
$$0.01 - 0.1 \sqrt{kPa}\ \text{min/l}.$$

2. Inhalation kit according to claim 1, **characterised in that** tiotropium is present in form of its chloride, bromide, iodide, methanesulphonate, para-toluenesulphonate or methyl sulphate, preferably in form of its bromide.

3. Inhalation kit according to claim 2, **characterised in that** tiotropium is present in form of its crystalline tiotropium bromide monohydrate.

4. Inhalation kit according to one of claims 1 to 3, wherein the inhaler is further **characterized in that** the deck has a reduced width in the range between deck and the housing near to the hinge, which forms the entrance slit for the air.

5. Inhaler suitable for the administration of an inhalable powder containing tiotropium in an amount of 0.001 to 5 %, in admixture with a physiologically acceptable excipient with an average particle size of between 10 to 500 μm, said inhaler comprises a housing, containing two windows, a deck in which there are air inlet ports and which is provided with a screen secured via a screen housing, an inhalation chamber connected to the deck on which there is a push button provided with two sharpened pins and movable counter to a spring, a mouthpiece which is connected to the housing, the deck and a cover via a spindle to enable it to be flipped open or shut, and three holes with diameters below 1 mm in the central region around the capsule chamber and underneath the screen housing and screen, and further **characterized in that** said inhaler displays a flow resistance of about $0.01 - 0.1 \sqrt{kPa}$ min/l.

6. Inhaler according to claim 5, wherein the inhaler is further **characterized in that** the deck has a reduced width in the range between deck and the housing near to the hinge, which forms the entrance slit for the air.

7. Inhaler according to claim 5 or 6 for use in a method for the treatment of airway diseases selected from asthma and COPD, wherein an inhalable powder containing tiotropium in an amount of 0.001 to 5 % in admixture with a physiologically acceptable excipient with an average particle size of between 10 to 500 μm is administered with the said inhaler.

8. Inhalable powder containing tiotropium in an amount of 0.001 to 5 % in admixture with a physiologically acceptable excipient with an average particle size of between 10 to 500 μm for use in a method for the treatment of airway diseases selected from asthma and COPD, wherein an inhaler acording to claim 5 or 6 is used for the administration of the inhalable powder.

**Patentansprüche**

1. Inhalationsset, bestehend aus einem Inhalator und einem inhalierbaren Pulver, enthaltend Tiotropium in einer Menge von 0,001 bis 5%, in Mischung mit einem physiologisch akzeptablen Hilfsstoff mit einer durchschnittlichen Partikelgröße zwischen 10 bis 500 μm, wobei der Inhalator ein Gehäuse umfasst, enthaltend zwei Fenster, ein Verdeck, in dem sich Lufteinlassoffnungen befinden und das mit einem Sieb versehen ist, gesichert bzw. befestigt über ein Siebgehäuse, eine Inhalationskammer, verbunden mit dem Verdeck, auf dem sich ein Betätigungsknopf befindet, vorgesehen mit zwei geschärften bzw. angespitzten Stiften und einem bewegbaren Zählwerk an einer Feder, einem Mundstück, das mit dem Gehäuse, dem Verdeck und einer Abdeckung über eine Spindel verbunden ist, um das Aufklappen oder Schließen zu ermöglichen, und drei Öffnungen mit Durchmessern unterhalb 1 mm im zentralen Bereich um die Kapselkammer und unter dem Siebgehäuse und dem Sieb, und weiterhin **dadurch gekennzeichnet, dass** der Inhalator einen Flusswiderstand von etwa $0{,}01$ bis $0{,}1 \sqrt{kPa}$ min/l zeigt.

2. Inhalationsset nach Anspruch 1, **dadurch gekennzeichnet, dass** Tiotropium in Form seines Chlorids, Bromids, Iodids, Methtmsulphonats, para-Toluolsulphonats oder Methylsulphats vorliegt, bevorzugt in Form des Bromids.

3. Inhalationsset nach Anspruch 2, **dadurch gekennzeichnet, dass** Tiotropium in Form des kristallinen Tiotropiumbromidmonohydrats vorliegt.

4. Inhalationsset nach einem der Ansprüche 1 bis 3, wobei der Inhalator weiterhin **dadurch gekennzeichnet ist, dass** das Verdeck eine reduzierte Breite im Bezeich zwischen dem Verdeck und dem Gehäuse in der Nähe des Gelenks bzw. Scharniers aufweist, das den Eintrittsschlitz bzw. -spalt für die Luft bildet.

5. Inhalator, der zur Verabreichung eines inhalierbaren Pulvers, enthaltend Tiotropium in einer Menge von 0,001 bis 5%, in Mischung mit einem physiologisch akzeptablen Hilfsstoff mit einer durchschnittlichen Partikelgröße zwischen 10 bis 500 μm geeignet ist, wobei der Inhalator ein Gehäuse umfasst, enthaltend zwei Fenster, ein Verdeck, in dem sich Lufteinlassöffnungen befinden und das mit einem Sieb versehen ist, gesichert bzw. befestigt über ein Siebgehäuse, eine Inhalationskammer, verbunden mit dem Verdeck, auf dem sich ein Betätigungsknopf befindet, versehen mit zwei geschärften bzw. angespitzten Stiften und einem bewegbaren Zählwerk an einer Feder, einem Mundstück, das mit dem Gehäuse, dem Verdeck und einer Abdeckung über eine Spindel verbunden ist, um das Aufklappen oder Schließen zu ermögliclien, und drei Öffnungen mit Durchmessern unterhalb 1 mm im zentralen Bereich un die Kapselkammer und unter dem Siebgehäuse und dem Sieb, und weiterhin **dadurch gekennzeichnet, dass** der Inhalator einen Flusswiderstand von etwa $0{,}01$ bis $0{,}1\ \sqrt{kPa}$ min/l zeigt.

6. Inhalator nach Anspruch 5, wobei der Inhalator weiterhin **dadurch gekennzeichnet ist, dass** das Verdeck eine reduzierte Breite im Bereich zwischen dem Verdeck und dem Gehäuse in der Nähe des Gelenks bzw. Scharniers aufweist, das den Eintrittsschlitz bzw. -spalt für die Luft bildet.

7. Inhalator nach Anspruch 5 oder 6 zur Verwendung in einem Verfahren zur Behandlung von Atemwegserkrankungen, ausgewählt aus Asthma und COPD, wobei ein inhalierbares Pulver, enthaltend Tiotropium in einer Menge von 0,001 bis 5%, in Mischung mit einem physiologisch akzeptablen Hilfsstoff mit einer durchschnittlichen Partikelgröße zwischen 10 bis 500 μm mit dem Inhalator verabreicht wird.

8. Inhalierbares Pulver, enthaltend Tiotropium in einer Menge von 0,001 bis 5%, in Mischung mit einem physiologisch akzeptablen Hilfsstoff mit einer durchschnittlichen Partikelgröße zwischen 10 bis 500 μm zur Verwendung in einem Verfahren zur Behandlung von Atemwegserkrankungen, ausgewählt aus Asthma und COPD, wobei ein Inhalator nach Anspruch 5 oder 6 zur Verabreichung des inhalierbaren Pulvers verwendet wird.

## Revendications

1. Kit d'inhalation constitué d'un inhalateur et d'une poudre inhalable contenant du tiotropium en une quantité de 0,001 à 5 %, mélangé avec un excipient physiologiquement acceptable avec une taille moyenne de particules comprise entre 10 et 500 μm, dans lequel l'inhalateur comprend un logement, contenant des fenêtres, un plateau dans lequel se trouvent deux orifices d'admission d'air et qui est doté d'une grille fixée via un logement de grille, une chambre d'inhalation raccordée au plateau sur laquelle se trouve un bouton poussoir doté de deux pointes acérées et mobile à l'encontre d'un ressort, un embout buccal qui est raccordé au logement, au plateau et à un capuchon via un axe pour permettre son ouverture ou sa fermeture par basculement, et trois trous ayant des diamètres inférieurs à 1 mm dans la région centrale autour de la chambre pour capsule et en dessous du logement de grille et de la grille, et en outre **caractérisé en ce que** ledit inhalateur présente une résistance au flux d'environ $0{,}01$ à $0{,}1\ \sqrt{kPa}$ min/L.

2. Kit d'inhalation selon la revendication 1, **caractérisé en ce que** le tiotropium est présent sous la forme de son chlorure, de son bromure, de son iodure, de son méthanesulfonate, de son para-toluènesulfonate ou de son méthyl sulfate, de préférence sous la forme de son bromure.

3. Kit d'inhalation selon la revendication 2, **caractérisé en ce que** le tiotropium est présent sous la forme de son bromure de tiotropium monohydraté cristallin.

4. Kit d'inhalation selon l'une quelconque des revendications 1 à 3, dans lequel l'inhalateur est en outre **caractérisé en ce que** le plateau a une largeur réduite dans la zone entre le plateau et le logement à proximité de la charnière,

formant la fente d'entrée pour l'air.

**5.** Inhalateur adapté pour l'administration d'une poudre inhalable contenant du tiotropium en une quantité de 0,001 à 5 %, mélangé avec un excipient physiologiquement acceptable avec une taille moyenne de particules de 10 à 500 μm, ledit inhalateur comprend un logement, contenant deux fenêtres, un plateau dans lequel se trouvent des orifices d'admission d'air et qui est doté d'une grille fixée via un logement de grille, une chambre d'inhalation raccordée au plateau sur laquelle se trouve un bouton poussoir doté de deux pointes acérées et mobile à rencontre d'un ressort, un embout buccal qui est raccordé au logement, au plateau et à un capuchon via un axe pour permettre son ouverture ou sa fermeture par basculement, et trois trous ayant des diamètres inférieurs à 1 mm dans la région centrale autour de la chambre pour capsule et en dessous du logement de grille et de la grille, et en outre **caractérisé en ce que** ledit inhalateur présente une résistance au flux d'environ 0,01 à 0,1 $\sqrt{kPa}$ min/L.

**6.** Inhalateur selon la revendication 5, dans lequel l'inhalateur est en outre **caractérisé en ce que** le plateau a une largeur réduite dans la zone entre le plateau et le logement à proximité de la charnière, qui forme la fente d'entrée pour l'air.

**7.** Inhalateur selon la revendication 5 ou 6, destiné à être utilisé dans une méthode de traitement de maladies des voies respiratoires choisies parmi l'asthme et la BPCO, dans lequel une poudre inhalable contenant du tiotropium en une quantité de 0,001 à 5 % en mélange avec un excipient physiologiquement acceptable avec une taille moyenne de particules comprise entre 10 et 500 μm est administrée avec ledit inhalateur.

**8.** Poudre inhalable contenant du tiotropium en une quantité de 0,001 à 5 % mélangé avec un excipient physiologiquement acceptable avec une taille moyenne de particules comprise entre 10 et 500 pm destinée à être utilisée dans une méthode de traitement de maladies des voies respiratoires choisies parmi l'asthme et la BPCO, dans laquelle l'inhalateur selon la ravendication 5 ou 6 est utilisé pour l'administration de la poudre inhalable.

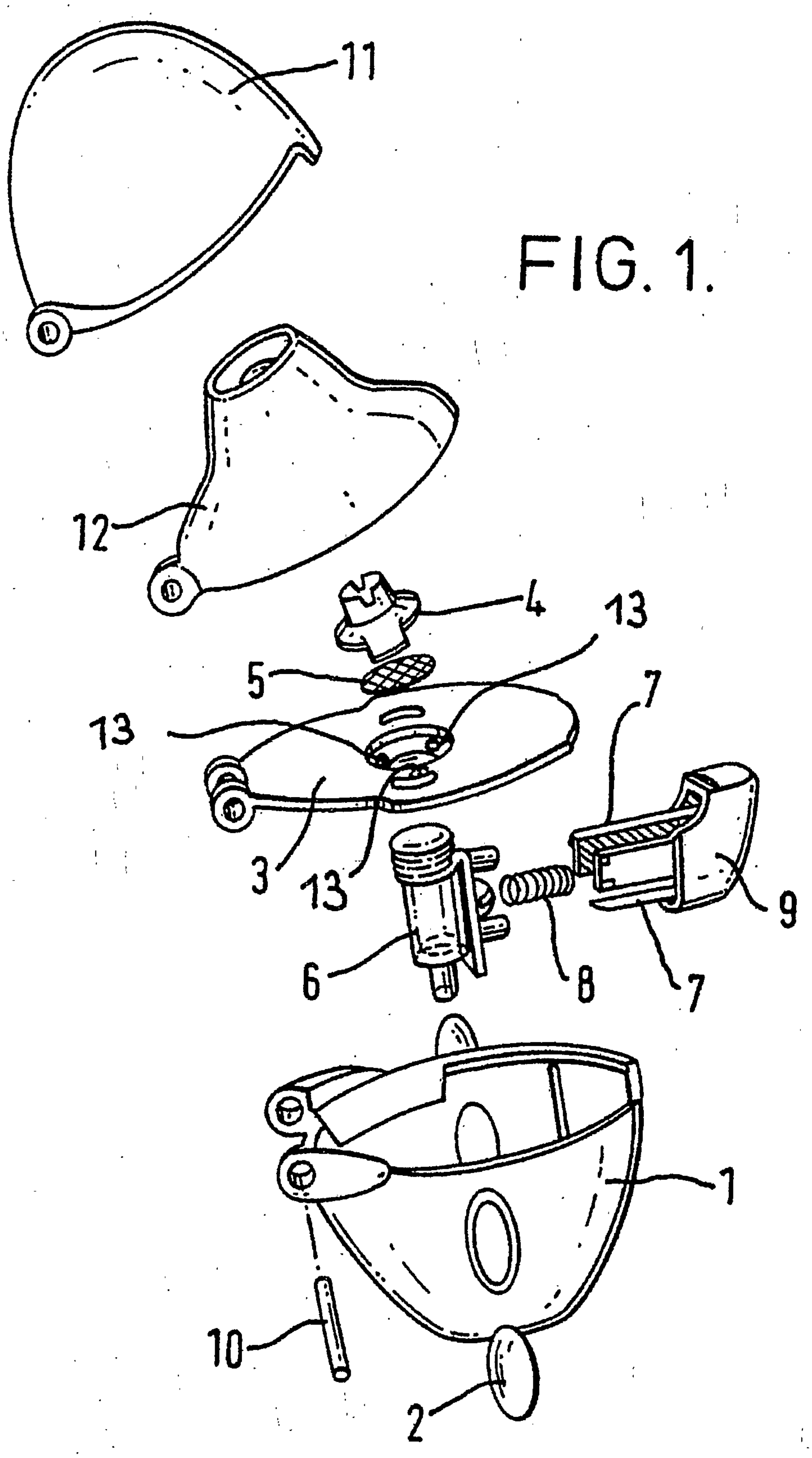


FIG. 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 418716 A1
- WO 0028979 A
- EP 1158970 A
- US 5590645 A
- US 4627432 A
- US 4524769 A
- US 5947118 A
- US D355029 S